Europäisches Patentamt

(19) European Patent Office

Office européen des brevets

(11) Publication number: **0 290 365**
**A2**

(12) **EUROPEAN PATENT APPLICATION**

(21) Application number: 88500043.0

(22) Date of filing: 04.05.88

(51) Int. Cl.⁴: **G 01 N 33/53**
**A 61 K 49/00**

(30) Priority: 06.05.87 ES 8701353

(43) Date of publication of application:
09.11.88 Bulletin 88/45

(84) Designated Contracting States:
AT BE CH DE FR GB GR IT LI NL SE

(71) Applicant: **Montero Iruzubieta, Jesus**
**Avda. Republica Argentina, 46**
**Sevilla (ES)**

**Lopez Elorza, Felix**
**Avda. Republica Argentina, 46**
**Sevilla (ES)**

**Mateo Canas, Joaquin**
**Avda. Republica Argentina, 46**
**Sevilla (ES)**

(72) Inventor: **Montero Iruzubieta, Jesus**
**Avda. República Argentina**
**E-46 Sevilla (ES)**

(74) Representative: **Lopez Medrano, Santiago**
**Gran Via, 40 -5.**
**E-28013 Madrid (ES)**

(54) **Procedure for the obtention of a product for the study of the liberation of anaphylactis mediators in primed cells.**

(57) A procedure for the obtention of a product for the study of the liberation of anaphilactic mediators in primed cells, characterized in comprising a two step reaction, one of preparation and another unchaining one, through the application of a prepared product and, immediately afterwards, of an unchaining product, being the preparing reactive a solution of albumin of human origin, coming from venous serum, stabilized and pasteurized during 10 hours at sixty degrees, whilst the unchaining is obtained with a viscous dissolvent, a proteic stabilizer, conserving and specific antigens, as per the types.

EP 0 290 365 A2

## Description

The present registration for patent of invention has the purpose, as its title indicates, of a procedure for the obtention of a product for the study of the liberation of anaphilactic mediators in primed cells, in accordance with the description made from same, which must be understood in its most ample sense and not limitatively.

The procedure which registration is preconized by means of the present writ assumes a solution absolutely original to the study of the liberation of anaphilactic mediators.

This procedure consists in a two step reaction, a preparation one and an unchaining one; in such a way that the exposure to the reactant is guaranteed at the place of the reaction.

### PRODUCT FOR THE PREPARATION REACTION

We will use a solution at 20% of albumin of human origin, coming from venous serum, stabilized and pasteurized during 10 hours at sixty degrees, as a preparing reactive.

### PRODUCT FOR THE UNCHAINING REACTION

For its obtention we will use:

- Viscous dissolvent such as: hydroxipropylmethylcellulose, methylcellulose, polivinylic alcohol, sodium hyaluronate, chondroitin sulphate, etc...in a quantity of 0,5 cc. to 15 cc.
- Proteic stabilizer: glycerin at a concentration variable between 0,1% at 10% as per the antigen.
- Preservers: such as phenol at 0,04%. Benzalchonium chloride, etc. In those cases in which you wish to keep under conditions of asepsis after its preparation. This element will not be necessary in those cases in which you do not wish to keep after its exposure to the environment.
- Specific antigens: in concentrations from 0 mgr/ml to 100 mgr/ml, as per the types of antigens.

List of antigens:
1 - Home dust
2 - Dermatofagoides Pteronnyssinus
3 - Dermatofagoides Farinas
4 - Different mixes of the foregoing
5 - Pollens
5.1 From wild graminaceous such as Phleum, Poa, Lolium, etc. not only individually but also mixed.
5.2 - Cultivated graminaceous, such as wheat, barley, rye, etc. not only individually but also mixed.
5.3 - Trees such as ash, oak, birch, elm, olive, false banana, etc. not only individually but also mixed.
5.4 - Herbs, such as mugwort, paristaria, etc. not only individually but also mixed.
5.5 - Flowers such as daises, rue, etc. not only individually but also mixed.
5.6 - Different mixes of the sub-groups of group 5.
6 - Fungi such as alternaria, asperigillus, penicillin, cladosporium, mucor, botrytia, Rhizopus, monilia, pullularia, curvularia, etc. not only individually but also mixed.
7 - Antigens of animal epitheliums such as dog, cat, horse, cow, sheep, rabbit, hampstead, birds feathers, etc., not only individually but also mixed.
8 - Trophoantigens such as milky proteins, meat proteins, of fish, of cereals, of egg, fruits, vegetables, etc. not only individually but also mixed.
9 - Colourings and preservers such as tartrazine, benzoate, etc., not only individually but also mixed.
10 - Chemotherapic compounds such as antibiotics, analgesics, anti-inflammatory products, anaesthesics, contrasts, etc. not only individually but also mixed.
11 - Microbial antigens, not only individually but also mixed.

The method for the obtention of the product object of this patent is obtained at the place of reaction in the following way: apply a volume between 10-50 microliters of the preparing product (albumin of human origin coming from venous serum, stabilized and pasteurized during 10 hours at sixty degrees) and immediately afterwards we will apply a volume of 10-50 microliters of the unchaining product, with the previously described margin of antigenic composition.

All the foregoing allows us to obtain an antigen prepared by an original method to be exposed in a controlled mode to a target cell and unchain in it a reaction of anaphilactic mediators.

Being sufficiently described the nature of the present invention, it is expressly made evident that any modification of detail that could be introduced, will be considered as included within same, as long as it does not alter or modify substantially its fundamental characteristics.

Finally, it is declared as of novelty and own invention, the following

## Claims

1.- PROCEDURE FOR THE OBTENTION OF A PRODUCT FOR THE STUDY OF THE LIBERATION OF ANAPHILACTIC MEDIATORS IN PRIMED CELLS, essentially characterized in that it comprises a two step reaction, one of preparation and another unchaining one, in such a way that the exposure to the reactant is guaranteed at the place of the reaction in the following way: applying a volume between 10 and 50 microliters of the preparing product (albumin of human origin coming from venous serum stabilized and pasteurized during 10 hours at sixty degrees) and immediately afterwards applying a volume from 10 to 50 microliters of the unchaining product.

2.- PROCEDURE FOR THE OBTENTION OF A PRODUCT FOR THE STUDY OF THE LIBERATION OF ANAPHILACTIC MEDIATORS IN PRIMED CELLS, as per claim 1, characterized in the fact that as a preparing reactive it will be used a solution at 20% of albumin of human origin coming from venous serum, stabilized and pasteurized during 10 hours at sixty degrees.

3.- PROCEDURE FOR THE OBTENTION OF A PRODUCT FOR THE STUDY OF THE LIBERATION OF ANAPHILACTIC MEDIATORS IN PRIMED CELLS, as per claim 1, characterized in the fact that for the obtention of the product for the unchaining reaction, it will be used viscous dissolvent, in a quantity of 0'5 cc. to 15 cc., proteic stabilizer (glycerin at a concentration variable between 0'1 at 10 per cent, as per the antigen), preserver (phenol at 0'04 %) and specific antigens, in concentrations from 0 mgr/ml to 100 mgr/ml, as per the types.

4.- PROCEDURE FOR THE OBTENTION OF A PRODUCT FOR THE STUDY OF THE LIBERATION OF ANAPHILACTIC MEDIATORS IN PRIMED CELLS.